(19) Europäisches Patentamt
European Patent Office
Office européen des brevets

(11) Veröffentlichungsnummer: **0 346 759 A2**

# (12) EUROPÄISCHE PATENTANMELDUNG

(21) Anmeldenummer: 89110349.1

(22) Anmeldetag: 08.06.89

(51) Int. Cl.4: C07J 9/00 , A61K 31/575 , //C07J31/00,C07J53/00, C07J17/00

Patentansprüche für folgende Vertragsstaaten: ES + GR.

(30) Priorität: 14.06.88 DE 3820177

(43) Veröffentlichungstag der Anmeldung: 20.12.89 Patentblatt 89/51

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Kreiser, Wolfgang Hermann, Prof. Dr.
Rehnocken 35b
D-5810 Witten-Herbede(DE)
Erfinder: Scharein, Peter, Dr.
Haenischstrasse 9
D-4600 Dortmund(DE)
Erfinder: Granzer, Ernold, Dr. Dr.
Falkensteiner Strasse 24
D-6233 Kelkheim (Taunus)(DE)

(54) **Cholestadienderivate, Verfahren zu ihrer Herstellung, ihre Verwendung als Arzneimittel mit HMG-CoA-Reduktase-hemmender Wirkung.**

(57) Verbindungen I

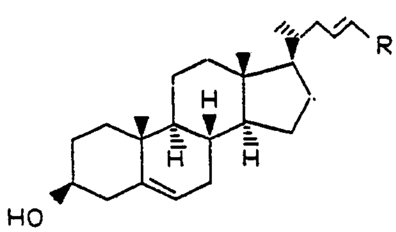

I

mit R gleich -COOX oder $CH_2OH$, wobei X Wasserstoff oder Alkyl bedeutet, weisen Cholesterin-senkende Wirkung auf. Sie werden durch veresterung der Verbindungen III

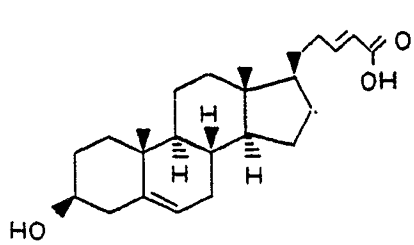

III

erhalten.

EP 0 346 759 A2

**Cholestadienderivate, Verfahren zu ihrer Herstellung, ihre Verwendung als Arzneimittel mit HMG-CoA-Reduktase-hemmender Wirkung**

Die Erfindung betrifft Cholestadienderivate, Verfahren zu ihrer Herstellung, ihre Verwendung als Arzneimittel mit HMG-CoA-Reduktase-hemmender Wirkung.

Es ist bekannt, aus Stigmasterol X das Stigmasteroltosylat IX herzustellen und dieses zum Stigmasterylmethylether VIII weiterumzusetzen. Aus diesem wird anschließend der literaturbekannte Aldehyd VII synthetisiert.

Über eine cholesterinsenkende Wirkung solcher Verbindungen ist jedoch bisher nichts bekannt geworden.

Es bestand deshalb das Bedürfnis, die bekannten Verbindung so abzuwandeln, daß hervorragende cholesterinsenkende Eigenschaften erzielt werden. Dies wurde erfindungsgemäß mit den Verbindungen I erreicht,

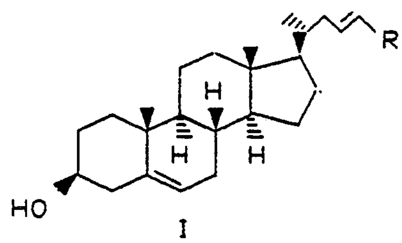

I

in denen R gleich -COOX oder $-CH_2OH$ ist, wobei X Wasserstoff oder $(C_1-C_8)$-Alkyl, geradkettig oder verzweigt, bedeutet.

Bevorzugt sind Verbindungen I mit X gleich $(C_1-C_4)$-Alkyl. Besonders bevorzugt sind Verbindungen I, worin X eine $CH_3$-Gruppe darstellt.

Ebenfalls bevorzugt sind Verbindungen I, worin R eine $-CH_2OH$-Gruppe bedeutet.

Die erfindungsgemäßen Verbindungen werden nach dem folgenden Reaktionsschema dargestellt:

Reaktionsschema:

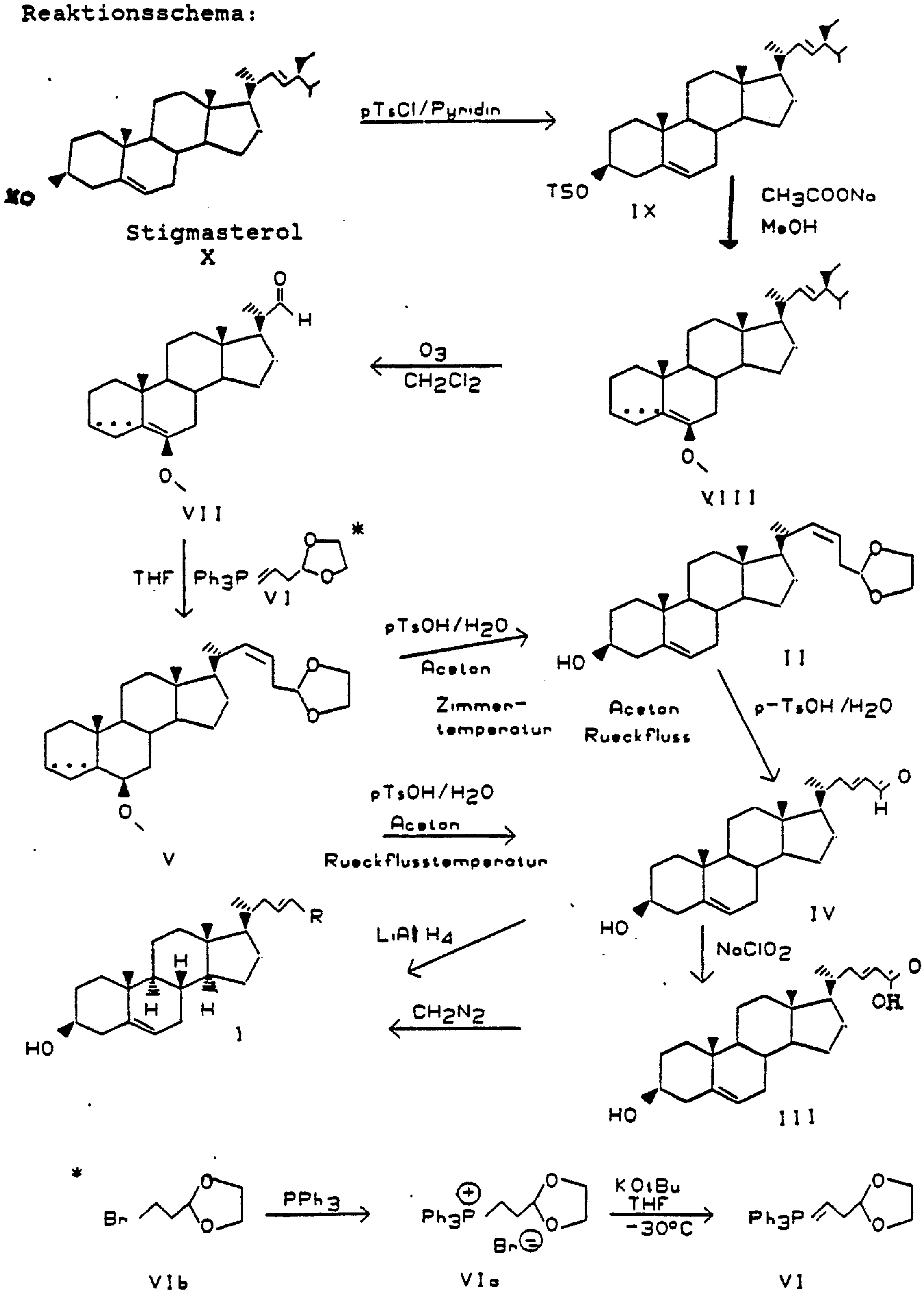


Stigmasterol
X
IX
VIII
VII
VI
V
II
IV
III
I
VIb
VIa
VI

Durch Reaktion von Stigmasterol X mit p-Toluolsulfonsäurechlorid in Pyridin bei Zimmertemperatur erhält man das Stigmasteroltosylat IX, das in wasserfreiem Methanol in Gegenwart von wasserfreiem Natriumacetat bei Rückflußtemperatur zum Stigmasterylmethylether VIII weiterumgesetzt wird (E. Mosettig, J.A.Steel J.Org.Chem. 28 (1963), 571-572). Die anschließende Ozonolyse des Methylethers der Formel VIII in wasserfreiem Methylenchlorid bei -78° C liefert nach Aufarbeitung den Aldehyd der Formel VII (R.F.N. Hutchins, M.F. Thompson, J.A. Svoboda, Steroids 15 (1970) 113-130). Aus dem Phosphoniumbromid der Formel VIa (hergestellt aus dem Dioxolan der Formel VIb in einem Lösungsmittelgemisch aus wasserfreiem Benzol und wasserfreiem Cyclohexan mit Triphenylphosphin bei Rückflußtemperatur) wird in wasserfreiem Tetrahydrofuran in Gegenwart von Kalium-t-butanolat als Base bei -10° C bis -25° C das Ylid der Formel VI erzeugt, das direkt mit hinzugefügtem Aldehyd der Formel VII bei 0° C zum Dioxolanderivat der Formel V weiterreagiert. Dieses liefert in einem Aceton/Wassergemisch nach Zugabe von p-Toluolsulfonsäure bei Rückflußtemperatur den Aldehyd der Formel IV.

Der Aldehyd der Formel IV läßt sich auch aus dem Dioxolan der Formel II in Gegenwart von p-Toluolsulfonsäure in einem Aceton/Wassergemisch bei Rückflußtemperatur herstellen. Das Dioxolan der Formel II wird durch Umsetzung des Dioxolans der Formel V mit p-Toluolsulfonsäure in einem Aceton/Wassergemisch bei Zimmertemperatur erhalten. Der Aldehyd der Formel IV wird anschließend in t-Butanol bei Zimmertemperatur mit Natriumchlorit zur Carbonsäure der Formel III oxidiert. Aus der Carbonsäure der Formel III wird in wasserfreiem Tetrahydrofuran mit einer etherischen Diazomethanlösung bei 0° C die Verbindung der Formel I mit R gleich -COOX, worin X eine $CH_3$-Gruppe bedeutet hergestellt.

Diese Verbindung I kann mit Lithiumaluminiumhydrid in wasserfreiem Tetrahydrofuran bei Rückflußtemperatur zur Verbindung der Formel I mit R gleich $CH_2OH$ reduziert werden.

Ein alternatives Verfahren zur Herstellung der Verbindung der Formel I mit R gleich $-CH_2OH$ besteht darin, den Aldehyd der Formel IV in wasserfreiem Ether mit Lithiumaluminiumhydrid bei Rückflußtemperatur zu reduzieren.

Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterin-Biosynthese (I.R. Sabine, 3-Hydroxy-3-methylglutaryl Coenzym A Reductase, CRC Press, 1983). Hohe Cholesterinspiegel werden mit einer Reihe von Erkrankungen wie z.B. koronarer Herzkrankeit oder Atherosklerose in Verbindung gebracht. Daher ist die Senkung erhöhter Cholesterinspiegel zur Vorbeugung und Behandlung solcher Erkrankungen ein therapeutisches Ziel. Ein Ansatzpunkt liegt in der Hemmung bzw. Verminderung der endogenen Cholesterinsynthese. Hemmstoffe der HMG-CoA-Reduktase blockieren die Cholesterin-Biosynthese auf einer frühen Stufe. Sie eignen sich daher zur Vorbeugung und Behandlung von Erkrankungen, die durch einen erhöhten Cholesterinspiegel verursacht werden. Eine Reduktion bzw. Verminderung der endogenen Synthese führt zu einer erhöhten Aufnahme von Cholesterin aus dem Plasma in die Zellen. Ein zusätzlicher Effekt läßt sich durch gleichzeitige Gabe Gallensäuren-bindender Stoffe wie Anionenaustauscher erzielen. Die erhöhte Gallensäurenausscheidung führt zu einer verstärkten Neusynthese und damit zu einem erhöhten Cholesterinabbau (M.S. Brown, P.T. Kovanen, J.L. Goldstein, Spektrum der Wissenschaften 1985 (1), 96). Die erfindungsgemäßen Verbindungen sind Hemmstoffe der HMG-CoA-Reduktase. Sie eignen sich daher zur Hemmung bzw. Verminderung der Cholesterin-Biosynthese und damit zur Vorbeugung oder Behandlung von Erkrankungen, die durch erhöhte Cholesterinspiegel verursacht werden, insbesondere koronare Herzkrankheit, Atherosklerose, Hypercholesterinämie, Hyperlipoproteinämie und ähnliche Erkrankungen.

Die Erfindung betrifft daher auch pharmazeutische Präparate auf Basis der Verbindungen der Formel I sowie die Verwendung dieser Verbindungen als Arzneimittel, insbesondere zur Behandlung der Hypercholesterinämie.

Die Verbindungen der Formel I werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 2500 mg, vorzugsweise jedoch in Dosisbereich 10 - 500 mg. Die erfindungsgemäßen Verbindungen können zur Anwendung kommen gelöst oder suspendiert in pharmakologisch unbedenklichen organischen Lösungsmitteln, wie ein- oder mehrwertigen Alkoholen, wie z.B. Ethanol oder Glycerin, in Triacetin, Ölen wie z.B. Sonnenblumenöl, Lebertran, Ethern, wie z.B. Diethylenglykoldimethylether oder auch Polyethern wie z.B. Polyethylenglykol, oder auch in Gegenwart anderer pharmakologisch unbedenklicher Polymerträger, wie z.B. Polyvinylpyrrolidon, oder anderen pharmazeutisch annehmbaren Zusatzstoffen wie Stärke, Cyclodextrin oder Polysacchariden. Ferner können die erfindungsgemäßen Verbindungen kombiniert werden mit Zusatzstoffen, die Gallensäuren binden, insbesondere nicht toxische, basische Anionenaustauscherharze, die Gallensäure in einer nichtresorbierbaren Form im Gastrointestinaltrakt binden.

**Prüfung auf Cholesterin-Biosynthese-Hemmung in Zellkulturen durch $^{14}$C-Präkursoreinbau in Cholesterin.**

Prinzip der Methoden

Monolayers von HEP G2-Zellen in lipoproteinfreiem Nährmedium wurden mit entsprechenden Konzentrationen der Prüfsubstanzen 1 Stunde vorinkubiert; nach Zugabe des $^{14}$C-markierten Präkursors $^{14}$C-Natriumacetat wurde die Inkubation für 3 Stunden fortgesetzt. Danach wurde ein Teil der Zellen bei vorheriger Zugabe eines internen Standards von $^{3}$H-Cholesterin alkalisch verseift. Die Lipide der verseiften Zellen wurden mit Chloroform-Methanol extrahiert. Dieses Lipidgemisch wurde nach Zusatz von Träger-Cholesterin präparativ dünnschichtchromatographisch getrennt, die Cholesterin-Bande nach dem Sichtbarmachen mit Jod-Dämpfen isoliert und die aus dem $^{14}$C-Präkursor gebildete Menge $^{14}$C-Cholesterins szintigraphisch bestimmt. In einem aliquoten Teil der Zellen wurde Zellprotein bestimmt, sodaß die in der Zeiteinheit/mg Zellprotein aus $^{14}$C-Präkursor gebildete Menge $^{14}$C-Cholesterins berechnet werden kann. Zum Vergleich für die Hemmwirkung eines zugesetzten Prüfpräparates diente die Lösungsmittelkontrolle, sodaß direkt die Hemmung der Cholesterin-Biosynthese bei einer bestimmten molaren Konzentration des Prüfpräparates im Medium angegeben werden kann. In aliquoten Anteilen der Zellkultur wurde die Intaktheit der Zellkulturen und die fehlende Zellschädigung durch Präparate-Einwirkung morphologisch (Lichtmikroskop) beurteilt.

Herstellung der Ausgangsmaterialien

**Beispiel 1**

3$\beta$-(p-Tosyloxy)-24(R)-ethyl-cholesta-5,22t-dien (IX)

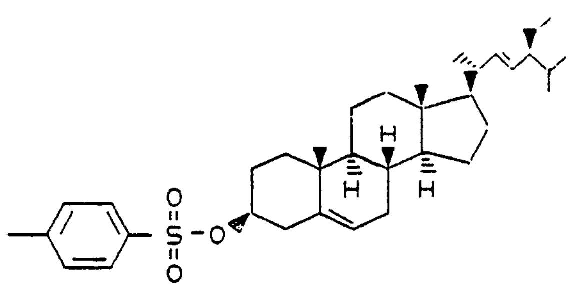

IX

Summenformel: $C_{36}H_{54}O_3S$ Molekulargewicht: 566,89 g/Mol

Zur Synthese des Stigmasterol-Tosylates IX ist es empfehlenswert, das zu verwendende p-Toluolsulfonsäurechlorid zuvor aus niedrig siedendem Petrolether (Kp: 35-60 °C) umzukristallisieren.

82,56 g (0,2 Mol) Stigmasterol X werden zusammen mit 99,12 g (0,52 Mol) p-Toluolsulfonsäurechlorid in 1120 ml absolutem Pyridin gelöst. Die Lösung wird 16 h im Dunkeln belassen und anschließend vorsichtig in 5,6 l einer eiskalten und gesättigten wäßrigen Natriumhydrogencarbonat-Lösung gegeben (Vorsicht: ggf. starkes Schäumen). Der anfallende farblose Niederschlag wird abgesaugt und gründlich mit eiskaltem Wasser und wenig eiskaltem Methanol gewaschen. Nach Trocknung im Vakuum wird aus absolutem Aceton umkristallisiert.

Ausbeute: 111,34 g (0,1964 Mol; 98,2 % d.Th.)
3$\beta$-(p-Tosyloxy)-24(R)-ethyl-cholesta-5,22t-dien IX
Farblose Nadeln Fp: 144-146 °C

| Elementar-Analyse | | |
|---|---|---|
| | berechnet | gefunden |
| C | 76,28 % | 76,0 % |
| H | 9,60 % | 9,5 % |
| X | 14,12 % | 14,5 % |

| IR-Spektrum (KBr-Preßling) | | |
|---|---|---|
| | Int. | Schwingung |
| 3010 $cm^{-1}$ | m | C-H Val.-Schwg. (arom.) |
| 2980 $cm^{-1}$ | s | C-H Val.-Schw. (aliph.) |
| 1590 $cm^{-1}$ | m | arom. Ring |
| 1360 $cm^{-1}$ | s | $-SO_2-O-$ |
| 1195 $cm^{-1}$ | s | $-SO_2-O-$ |
| 1175 $cm^{-1}$ | s | $-SO_2-O-$ |
| 955 $cm^{-1}$ | s | -C=CH- (Ring) |
| 840 $cm^{-1}$ | s | Aromat (p-substituiert) |
| 680 $cm^{-1}$ | s | -CH=CH- (trans, Seitenkette) |

| $^{13}C$-NMR-Spektrum (75,47 MHz) in $CDCl_3$ | | | |
|---|---|---|---|
| 38,9 | ppm t C-1 | 12,0 | ppm q C-18 |
| 28,6 | ppm t C-2 | 19,0 | ppm q C-19 |
| 82,3 | ppm d C-3 | 40,4 | ppm d C-20 |
| 36,9 | ppm t C-4 | 21,06 | ppm q C-21 |
| 138,8 | ppm s C-5 | 138,2 | ppm d C-22 |
| 123,4 | ppm d C-6 | 129,3 | ppm d C-23 |
| 31,80 | ppm t C-7 | 51,2 | ppm d C-24 |
| 31,83 | ppm d C-8 | 31,78 | ppm d C-25 |
| 49,9 | ppm d C-9 | 19,1 | ppm q C-26* |
| 36,35 | ppm s C-10 | 21,2 | ppm q C-27* |
| 20,98 | ppm t C-11 | 25,4 | ppm t C-28 |
| 39,6 | ppm t C-12 | 12,2 | ppm q C-29 |
| 42,2 | ppm s C-13 | 144,3 | ppm s C-1′ |
| 56,7 | ppm d C-14 | 127,6 | ppm d C-2′, C-6′ |
| 24,3 | ppm t C-15 | 129,7 | ppm d C-3′, C-5′ |
| 28,8 | ppm t C-16 | 134,8 | ppm s C-4′ |
| 55,9 | ppm d C-17 | 21,6 | ppm q C-7′ |

| Optische Drehwerte (c = 1,6613 in $CHCl_3$; T = 25° C) | | |
|---|---|---|
| λ | α | [α] |
| 589 nm | -0,753 | -45,3 |
| 578 nm | -0,777 | -46,8 |
| 546 nm | -0,889 | -53,5 |
| 436 nm | -1,540 | -92,7 |
| 365 nm | -2,482 | -149,4 |

| $^{1}H$-NMR-Spektrum (300,13 MHz) in $CDCl_3$ | | | | | |
|---|---|---|---|---|---|
| 0,64 | ppm | s | 3 | H | $-CH_3$ (C-18) |
| 0,78 | ppm | d | 6 | H | $-CH_3$ (C-26, C-27) $^{3}J$: 1,2 Hz |
| 0,81 | ppm | t | 3 | H | $-CH_3$ (C-29) $^{3}J$: 6.1 Hz |
| 0,97 | ppm | s | 3 | H | $-CH_3$ (C-19) |
| 1,01 | ppm | d | 3 | H | $-CH_3$ (C-21) $^{3}J$: 6.5 Hz |
| 0,76 - 2,51 | ppm | m | 25 | H | nicht zugeordnete Protonen |
| 2,44 | ppm | s | 3 | H | $-CH_3$ (Aromat) |
| 4,39 - 4,26 | ppm | m | 1 | H | 3 -H |
| 5,01 | ppm | dd | 1 | H | =CH- (C-22) $^{3}J$: 14,8 Hz $^{3}J$: 6,6 Hz |
| 5,14 | ppm | dd | 1 | H | =CH- (C-23) $^{3}J$: 14,8 Hz $^{3}J$: 6,8 Hz |
| 5,32 - 5,28 | ppm | m | 1 | H | =CH- (C-6) |
| 7,33 | ppm | d | 2 | H | =CH- (C-2′, C-6′) $^{3}J$: 8,5 Hz |
| 7,79 | ppm | d | 2 | H | =CH- (C-3′, C-5′) $^{3}J$: 8,5 Hz |

| Massenspektrum (MAT, 70 eV, T: 125° C) | | |
|---|---|---|
| $M^+$ (ber.): 566,3794 g/Mol | | |
| Masse | I(rel.) | Fragment |
| m/e = 566 | 3,2 % | $M^+$ |
| m/e = 551 | 4,3 % | $M^+$ $-CH_3$ |
| m/e = 536 | 2,6 % | $M^+$ -2x $CH_3$ |
| m/e = 402 | 24,4 % | $M^+$ $-C_7H_7OS$ |
| m/e = 395 | 64,3 % | $M^+$ $-C_7H_7O_3S$ |
| m/e = 394 | 33,6 % | $M^+$ $-C_7H_8O_3S$ |
| m/e = 352 | 41,1 % | $M^+$ $-C_7H_7O_3S$, $-CH(CH_3)_2$ |
| m/e = 284 | 27,4 % | $M^+$ $-C_7H_7O_3S$, $-CH=CH-CH(C_2H_5)CH(CH_3)_2$ |
| m/e = 137 | 100,0 % | $C_7H_7OS^+$ |
| m/e = 91 | 64,0 % | Tropylium-Ion |

**Beispiel 2**

3α,5α-Cyclo-6β-methoxy-24(R)-ethyl-cholest-22t-en (VIII)

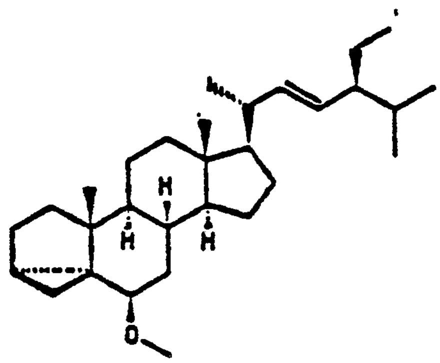

VIII

Summenformel: $C_{30}H_{50}O$  Molekulargewicht: 426,73 g/Mol

56,70 g (0,10 Mol) 3β-(p-Tosyloxy)-24(R)-ethyl-cholesta-5,22t-dien IX werden zusammen mit 61,5 g (0.75 Mol) wasserfreiem Natriumacetat in 2250 ml absolutem Methanol suspendiert. Nach 3,5 h Erhitzen unter Rückfluß löst sich die Suspension vollständig auf. Zur Beendigung der Reaktion wird das Methanol abdestilliert und der verbleibende weiße Rückstand mit 1000 ml Wasser und 1000 ml Essigester versetzt. Die organische Phase wird abgetrennt, mit Natriumhydrogencarbonat-Lösung und Wasser gewaschen und über Magnesiumsulfat getrocknet. Nach Abziehen des Lösungsmittels im Vakuum erhält man das Produkt als farbloses zähes Öl (40,25 g), das für die weiteren Reaktionen ausreichend rein ist. Die Substanz kristallisiert bei längerem Stehenlassen in Form von farblosen Rhomben.

Ausbeute: 40,25 g (0,094 Mol; 94 % d.Th.)

3α,5β-Cyclo-6β-methoxy-24(R)-ethyl-cholest-22t-en VIII

Eine Reinigung zur analytische Charakterisierung kann durch präparative Schichtchromatographie [PSC] (Kieselgel; Cyclohexan/Essigester : 20/1) ($R_f$: 0,81-0,88) mit anschließender Umkristallisation aus abs. Aceton erfolgen. Man erhält farblose Nadeln mit einem Schmelzpunkt von 56-58 °C.

| IR-Spektrum (KBr-Preßling) | | |
|---|---|---|
| | Int. | Schwingung |
| 2960 $cm^{-1}$ | s | C-H Val.-Schwg. (aliph.) |
| 2880 $cm^{-1}$ | m | C-H Val.-Schwg. (aliph.) |
| 1450 $cm^{-1}$ | s | $-CH_2-$, $-CH_3$ Def.-Schwg. |
| 1380 $cm^{-1}$ | s | $-CH_3$ Def.-Schwg. |
| 1095 $cm^{-1}$ | s | C-O-C |
| 960 $cm^{-1}$ | s | C=C (trans) |

| Optische Drehwerte (c = 2,91 in $CHCl_3$; T = 25 °C) | | |
|---|---|---|
| λ | α | [α] |
| 589 nm | +0,074 | 2,5 |
| 578 nm | +0,077 | 2,7 |
| 546 nm | +0,087 | 3,0 |
| 436 nm | +0,145 | 5,1 |
| 365 nm | +0,236 | 8,1 |

| $^1$H-NMR-Spektrum (300,13 MHz) in $CDCl_3$ | | | | | | |
|---|---|---|---|---|---|---|
| 0,43 | ppm | dd | 1 | H | -CHH- (C-4, äq.) | $^2$J: 8,1 Hz<br>$^3$J: 5,0 Hz |
| 0,66 | ppm | dd | 1 | H | -CHH- (C-4, ax.) | $^2$J: 8,1 Hz<br>$^3$J: 3,9 Hz |
| 0,74 | ppm | s | 3 | H | $-CH_3$ (C-18) | |
| 0,78 | ppm | d | 6 | H | $-CH_3$ (C-26, C-27) | |
| 0,805 | ppm | s | 3 | H | $-CH_3$ (C-19) | |
| 0,81 | ppm | t | 3 | H | $-CH_3$ (C-29) | $^3$J: 6,2 Hz |
| 0,85 | ppm | d | 3 | H | $-CH_3$ (C-21) | $^3$J: 6,5 Hz |
| 0,79 - 2,09 | ppm | m | 30 | H | nicht zugeordnete Protonen | |
| 2,77 | ppm | dd | 1 | H | $-CH(OCH_3)$ (C-6) | $^3$J: 2,7 Hz<br>$^3$J: 2,8 Hz |
| 3,32 | ppm | s | 3 | H | $-O-CH_3$ | |
| 5,01 | ppm | dd | 1 | H | -CH = CH- (C-23) | $^3$J: 15,1 Hz<br>$^3$J: 8,6 Hz |
| 5,15 | ppm | dd | 1 | H | -CH = CH-(C-22) | $^3$J: 15,1 Hz<br>$^3$J: 8,6 Hz |

| Massenspektrum (MAT, 70 eV, T: 80 °C) | | |
|---|---|---|
| $M^+$(ber.): 426,3862 g/Mol | | |
| Masse | I(rel.) | Fragment |
| m/e = 426 | 100,0 % | $M^+$ |
| m/e = 411 | 12,7 % | $M^+$ $-CH_3$ |
| m/e = 395 | 39,2 % | $M^+$ $-OCH_3$ |
| m/e = 383 | 6,2 % | $M^+$ $-CH(CH_3)_2$ |
| m/e = 380 | 9,8 % | $M^+$ $-CH_3$, $-OCH_3$ |
| m/e = 371 | 13,6 % | $M^+$ $-CH_2CH_2CHCH_2$ (A-Ring-Fragment) |
| m/e = 315 | 20,3 % | $M^+$ $-CH=CH-CH(CH_2CH_3)CH(CH_3)_2$ |
| m/e = 284 | 44,6 % | $M^+$ $-CH=CH-CH(CH_2CH_3)CH(CH_3)_2$, $-OCH_3$ |

| $^{13}$C-NMR-Spektrum (75,47 MHz) in $CDCl_3$ | | | |
|---|---|---|---|
| 35,1 | ppm t C-1 | 29,0 | ppm t C-16 |
| 33,4 | ppm t C-2 | 56,1 | ppm d C-17 |
| 21,5 | ppm d C-3 | 12,5 | ppm q C-18 |
| 13,1 | ppm t C-4 | 19,0 | ppm q C-19 |
| 35,3 | ppm s C-5 | 40,5 | ppm d C-20 |
| 82,4 | ppm d C-6 | 21,1 | ppm q C-21 |
| 24,3 | ppm t C-7 | 138,4 | ppm d C-22 |
| 30,5 | ppm d C-8 | 129,2 | ppm d C-23 |
| 48,1 | ppm d C-9 | 51,3 | ppm d C-24 |
| 43,45 | ppm s C-10 | 31,9 | ppm d C-25 |
| 22,8 | ppm t C-11 | 19,3 | ppm q C-26* |
| 40,2 | ppm t C-12 | 21,2 | ppm q C-27* |
| 42,7 | ppm s C-13 | 25,4 | ppm t C-28 |
| 56,6 | ppm d C-14 | 12,3 | ppm q C-29 |
| 25,0 | ppm t C-15 | 56,5 | ppm q $O-CH_3$ |

| $^{13}C$-$^{1}H$-NMR-Korrelations-Spektrum ($CDCl_3$) | | | | | |
|---|---|---|---|---|---|
| C-Atom | $^{13}C$ (ppm) | $^{1}H$ (ppm) | C-Atom | $^{13}C$ (ppm) | $^{1}H$ (ppm) |
| 1 t | 35,1 | ca. 1,1/1,9 | 16 t | 29,0 | ca. 1,52 |
| 2 t | 33,4 | ca. 1,5 | 17 d | 56,1 | ca. 1,18 |
| 3 d | 21,5 | ca. 0,92 | 18 q | 12,5 | 0,74 |
| 4 t | 13,1 | 0,43/0,66 | 19 q | 19,0 | 0,805 |
| 5 s | 35,3 | - | 20 d | 40,5 | ca. 2,05 |
| 6 d | 82,4 | 2,77 | 21 q | 21,1 | 0,85 |
| 7 t | 24,3 | 1,57 | 22 d | 138,4 | 5,15 |
| 8 d | 30,5 | ca. 1,75 | 23 d | 129,2 | 5,01 |
| 9 d | 48,1 | ca. 0,82 | 24 d | 51,3 | ca. 1,52 |
| 10 s | 43,4 | - | 25 d | 31,9 | ca. 1,55 |
| 11 t | 22,8 | ca. 1,92 | 26 q* | 19,3 | 1,02 |
| 12 t | 40,2 | ca. 1,98 | 27 q* | 21,2 | 1,02 |
| 13 s | 42,7 | - | 28 t | 25,4 | ca. 1,3 |
| 14 d | 56,6 | ca. 1,05 | 29 q | 12,3 | 0,81 |
| 15 t | 25,0 | ca. 1,2 | $-OCH_3$ q | 56,5 | 3,32 |

| Elementar-Analyse | | |
|---|---|---|
| | berechnet | gefunden |
| C | 84,44 % | 84,3 % |
| H | 11,81 % | 11,9 % |
| X | 3,75 % | 3,8 % |

**Beispiel 3**

3α,5α-Cyclo-6β-methoxy-pregnan-20(S)-carbaldehyd (VII)

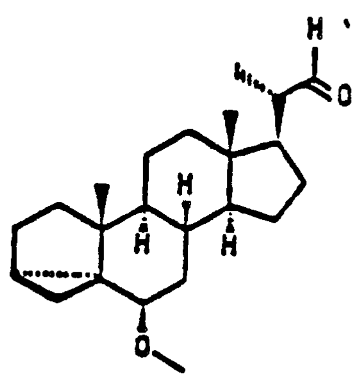

VII

**Summenformel: $C_{23}H_{36}O_2$ Molekulargewicht: 344,54 g/Mol**

8,05 g (ca. 0,018 Mol) des rohen 3α, 5α-Cyclo-6β-methoxy-24(R)-ethyl-cholest-22t-ens VIII werden in

300 ml absolutem Methylenchlorid gelöst und auf -78° C gekühlt. Durch diese Lösung leitet man bis zur deutlichen Blaufärbung Ozon (Fischer Ozongenerator; $O_2$ ca. 40 l/h; U = 180 V; I = 2,4 A; Ozongehalt ca. 60 mg/l entspr. 0,05 Mol/h). Nach Beendigung der Ozonolyse erfolgt Zugabe von 14,8 ml (12,5 g; 0,20 Mol) Dimethylsulfid bei -78° C. Anschließend läßt man das Reaktionsgemisch sich innerhalb von 3 Stunden auf Zimmertemperatur erwärmen. Nun wird es nacheinander mit 200 ml Wasser, 200 ml gesättigter Natriumhydrogencarbonat-Lösung und 100 ml gesättigter Natriumchlorid-Lösung gewaschen und über wasserfreiem Natriumsulfat getrocknet. Nach Abfiltrieren des Trockenmittels wird das Lösungsmittel am Rotationsverdampfer bei ca. 12 Torr abdestilliert. Es schließt sich eine 45-minütige Trocknung bei 0,1 Torr an.

Man erhält 6,0 g (ca. 0,017 Mol; ca. 94 %) rohen 3α,5α-Cyclo-6β-methoxy-pregnan-20(R)-carbaldehyd VII als farbloses zähes Öl. Der Aldehyd neigt zur Isomerisierung am C-20 und zur Oxidation am C-21, so daß eine sofortige Weiterverarbeitung erfolgen sollte. Aus dem gleichen Grund ist eine Reinigung nicht empfehlenswert; eine Charakterisierung erfolgte nur NMR-spektroskopisch am relativ reinen Rohprodukt.

Ein alternatives Aufarbeitungsverfahren besteht in der Zugabe von 9,4 g Zinkstaub, 23,5 g Eisessig und 15,0 g wasserfreiem Calciumchlorid nach erfolgter Ozonolyse. Nach einstündiger Reaktion wird das Reaktionsgemisch filtriert und mit 200 ml eiskaltem Wasser und 100 ml Natriumhydrogencarbonat säurefrei gewaschen; ein Ausschütteln mit 50 ml Wasser und 50 ml gesättigter Natriumchlorid-Lösung schließt sich an. Die Methylenchlorid-Lösung trocknet man über wasserfreiem Natriumsulfat; anschließend entfernt man das Lösungsmittel unter vermindertem Druck (ca. 12 Torr) am Rotationsverdampfer. Zum vollständigen Entfernen des Lösungsmittels trocknet man noch etwa 45 min im Vakuum bei 0,1 Torr. Bei gleicher Ausbeute fällt hier das Rohprodukt etwas reiner an.

| $^1$H-NMR-Spektrum (300,13 MHz) in $CDCl_3$ | | | | | |
|---|---|---|---|---|---|
| 0,45 | ppm | dd | 1 | H | -CHH- (4-H äq.) $^2$J: 8,1 Hz $^3$J: 5,2 Hz |
| 0,68 | ppm | dd | 1 | H | -C$\overline{H}$H- (4-H ax.) $^2$J: 8,1 Hz $^3$J: 4,0 Hz |
| 0,77 | ppm | s | 3 | H | -C$\overline{H}_3$ (C-18) |
| 1,02 | ppm | s | 3 | H | -$CH_3$ (C-19) |
| 1,12 | ppm | d | 3 | H | -$CH_3$ (C-21) $^3$J: 6,8 Hz |
| 0,71 - 2,11 | ppm | m | 19 | H | nicht zugeordnete Protonen |
| 2,28 - 2,44 | ppm | m | 1 | H | -CH- (C-20) |
| 2,78 | ppm | m | 1 | H | -CH($OCH_3$) |
| 3,33 | ppm | s | 3 | H | -O$\overline{C}H_3$ |
| 9,58 | ppm | d | 1 | H | -CHO $^3$J: 3,2 Hz |

| $^{13}$C-NMR-Spektrum (75,47 MHz) in $CDCl_3$ | | | |
|---|---|---|---|
| 35,1 | ppm t C-1 | 43,4 | ppm s C-13 |
| 33,4 | ppm t C-2 | 55,8 | ppm d C-14 |
| 21,5 | ppm d C-3 | 24,0 | ppm t C-15 |
| 13,1 | ppm t C-4 | 27,2 | ppm t C-16 |
| 35,2 | ppm s C-5 | 52,6 | ppm d C-17 |
| 82,3 | ppm d C-6 | 12,4 | ppm q C-18 |
| 25,0 | ppm t C-7 | 19,3 | ppm q C-19 |
| 30,5 | ppm d C-8 | 42,1 | ppm d C-20 |
| 48,0 | ppm d C-9 | 17,1 | ppm q C-21 |
| 43,0 | ppm d C-10 | 205,2 | ppm d C-22 |
| 22,7 | ppm t C-11 | 56,6 | ppm d -O-$CH_3$ |
| 40,0 | ppm t C-12 | | |

**Beispiel 4**

3α,5α-Cyclo-6β-methoxy-24-(1′,3′-dioxolan-2′-yl)-chol-22c-en (V)

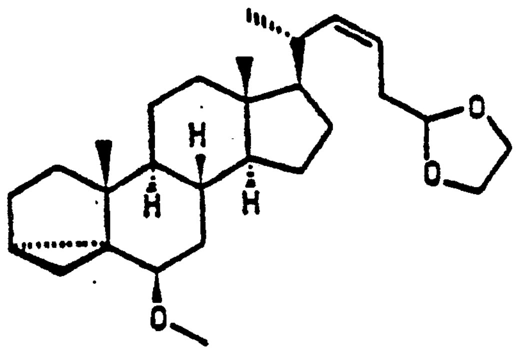

V

Summenformel: $C_{28}H_{44}O_3$ Molekulargewicht: 428,66 g/Mol

13,305 g (0,03 Mol) trockenes und fein pulverisiertes 2-(1′,3′-Dioxolan-2′-yl)-ethyl-triphenylphosphoniumbromid (VIa) werden in 100 ml wasserfreiem THF suspendiert und bei -10° C langsam 60,0 ml mit einer 0,5 molaren Lösung von Kalium-t-butanolat (0,03 Mol) in wasserfreiem THF versetzt. Die orangefarbene Ylid-Lösung rührt man bei -25° C maximal 30 min. und addiert bei dieser Temperatur eine Lösung von frisch hergestelltem 6,89 g (0,02 Mol) 3α,5α-Cyclo-6β-methoxy-pregnan-20(S)-carbaldehyd (VII) in 100 ml wasserfreiem THF. Nach erfolgter Zugabe erwärmt man auf 0° C und rührt 6 h bei dieser Temperatur. Zur Aufarbeitung gibt man zur resultierenden Suspension 50 ml eiskaltes Wasser und 100 ml Ether; nach Abtrennen der organischen Phase und Abziehen des Lösungsmittels wird der verbleibende hellgelbe Rückstand mindestens 4 mal mit ingesamt 500 ml Pentan extrahiert. Nachdem die Pentanlösung über wasserfreiem Magnesiumsulfat getrocknet und anschließend filtriert worden ist, entfernt man das Lösungsmittel zunächst unter vermindertem Druck (ca. 12 Torr) am Rotationsverdampfer und danach im Vakuum bei 0,1 Torr.

Man erhält ca. 7,4 g des rohen Kondensations-Produktes (V), das für die meisten Folgereaktionen ausreichend rein ist. Eine analytische Probe erhält man durch PSC (Kieselgel; Cyclohexan/Essigester : 4/l; $R_f$: 0,56-0,68). Das Produkt fällt als zähes Öl an; der Anteil an reinem cis-Isomer ist größer 90 %.

| IR-Spektrum (KBr-Preßling) | | |
|---|---|---|
| | Int. | Schwingung |
| 2990 $cm^{-1}$ | s | C-H Val.-Schwg. |
| 1710 $cm^{-1}$ | s | -CH = CH- (cis) |
| 1650 $cm^{-1}$ | m | Cyclopropan-Ring |
| 1430 $cm^{-1}$ | s | $-CH_3$, $-CH_2-$ |
| 790 $cm^{-1}$ | m | = CH- |

| $^1$H-NMR-Spektrum (300,13 MHz) in $CDCl_3$ | | | | | |
|---|---|---|---|---|---|
| 0,44 | ppm | dd | 1 | H | -CHH- (C-4, äq.) $^2$J: 8,0 Hz $^3$J: 5,0 Hz |
| 0,66 | ppm | dd | 1 | H | -CHH- (C-4, ax.) $^2$J: 8,0 Hz $^3$J: 3,9 Hz |
| 0,75 | ppm | s | 3 | H | $-CH_3$ (C-18) |
| 0,97 | ppm | d | 3 | H | $-CH_3$ (C-21) $^3$J: 6,5 Hz |
| 1,02 | ppm | s | 3 | H | $-CH_3$ (C-19) |
| 0,73 - 2,50 | ppm | m | 25 | H | nicht zugeordnete Protonen |
| 2,77 | ppm | m | 1 | H | -CH- (C-6) |
| 3,32 | ppm | s | 3 | H | $-OCH_3$ |
| 3,82 - 4,01 | ppm | m | 4 | H | $-O-CH_2CH_2-O-$ |
| 4,86 | ppm | t | 1 | H | $-CH-O-CH_2CH_2-O-$ |
| 5,21 - 5,38 | ppm | m | 2 | H | -CH=CH- |

| Optische Drehwerte (c = 5,3238 in $CHCl_3$; T = 25° C) | | |
|---|---|---|
| λ | α | [α] |
| 589 nm | +0,710 | +13,3 |
| 578 nm | +0,739 | +13,9 |
| 546 nm | +0,838 | +15,7 |
| 436 nm | +1,394 | +26,2 |
| 365 nm | +2,101 | +39,5 |

| $^{13}$C-NMR-Spektrum (75,47 MHz) in $CDCl_3$ | | | |
|---|---|---|---|
| 35,05 | ppm t C-1 | 24,0 | ppm t C-15 |
| 33,2 | ppm t C-2 | 28,0 | ppm t C-16 |
| 21,3 | ppm d C-3 | 56,35 | ppm d C-17 |
| 13,0 | ppm t C-4 | 12,4 | ppm q C-18 |
| 34,9 | ppm s C-5 | 19,2 | ppm q C-19 |
| 82,2 | ppm d C-6 | 34,4 | ppm d C-20 |
| 24,8 | ppm t C-7 | 20,4 | ppm q C-21 |
| 30,3 | ppm d C-8 | 139,5 | ppm d C-22 |
| 47,9 | ppm d C-9 | 119,0 | ppm d C-23 |
| 43,2 | ppm q C-10 | 32,5 | ppm t C-24 |
| 22,6 | ppm t C-11 | 104,0 | ppm d C-25 |
| 40,0 | ppm t C-12 | 56,3 | ppm q $-O-CH_3$ |
| 42,6 | ppm q C-13 | 64,7 | ppm t $-O-CH_2-CH_2-O$ |
| 55,9 | ppm d C-14 | | |

| Elementar-Analyse | | |
|---|---|---|
| | berechnet | gefunden |
| C | 78,46 % | 78,6 % |
| H | 10,35 % | 10,5 % |
| X | 11,19 % | 10,9 % |

Massenspektrum (MAT, 70 eV, T: 80°C)

$M^+$(ber.): 428,3290 g/Mol

| Masse | I(rel.) | Fragment |
| --- | --- | --- |
| m/e = 428 | 3,9 % | $M^+$ |
| m/e = 413 | 6,6 % | $M^+$ -$CH_3$ |
| m/e = 397 | 8,0 % | $M^+$ -$OCH_3$ |
| m/e = 382 | 10,1 % | $M^+$ -$CH_3$, -$OCH_3$ |
| m/e = 355 | 100,0 % | $M^+$ - 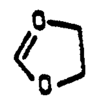 |
| m/e = 343 | 27,2 % | $M^+$ - 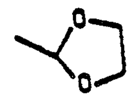 |
| m/e = 315 | 64,8 % | $M^+$ - 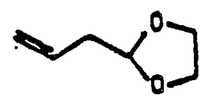 |
| m/e = 113 | 58,7 % | 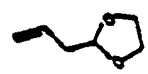 |

**Beispiel 5**

3β-Hydroxy-24-(1′,3′-dioxolan-2′-yl)-chola-5,22c-dien

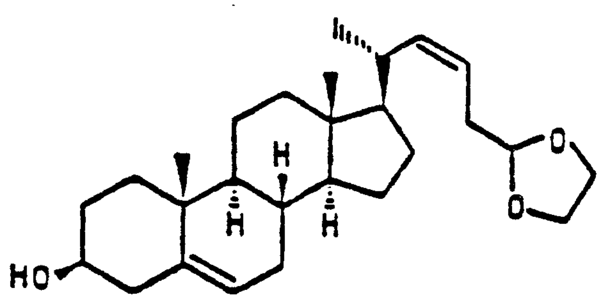

II

**Summenformel:** $C_{27}H_{42}O_3$ **Molekulargewicht:** 414,63 g/Mol

Die selektive säurekatalysierte Öffnung des Cyclopropansystems im Steroid-A-Ring gelingt durch 20-stündige Umsetzung bei Zimmertemperatur von 4,29 g (0,01 Mol) 3α,5α-Cyclo-6β-methoxy-24-(1′,3′-dioxolan-2′-yl)-chol-22c-en (V) mit 1,4 g (7,0 mMol) p-Toluolsulfonsäure in einem Lösungsmittelgemisch aus 150 ml Aceton und 20 ml Wasser. Nach Beendigung der Reaktion wird das Lösungsmittel bei Zimmertemperatur im Vakuum auf 20 % seines Volumens eingeengt, in Eiswasser gegossen und mit Essigester

extrahiert. Die Essigester-Lösung wird abgetrennt und nacheinander mit 100 ml Wasser, 50 ml gesättigter Natriumhydrogencarbonat-Lösung, 50 ml Wasser und 50 ml gesättigter Natriumchlorid-Lösung gewaschen. Nach Trocknung der organischen Lösung über wasserfreiem Magnesiumsulfat wird abfiltriert; man entfernt das Lösungsmittel unter vermindertem Druck (12 Torr) am Rotationsverdampfer und anschließend bei 0,1 Torr im Vakuum. Das erhaltene zähe Rohprodukt wird mittels PSC (Kieselgel; Cyclohexan/Essigester : 2/l; $R_f$: 0,28-0,37) gereinigt.
Ausbeute: 3,6 g (8,64 mMol, 85 % d.Th.) 3β-Hydroxy-24-(1′, 3′dioxolan-2′-yl)-chola-5,22c-dien (II)
Fp: 90-91 °C (nach PSC)

| Elementar-Analyse | | |
|---|---|---|
| | berechnet | gefunden |
| C | 78,21 % | 78,0 % |
| H | 10,21 % | 10,0 % |
| X | 11,58 % | 12,0 % |

| IR-Spektrum (KBr-Preßling) | | |
|---|---|---|
| | Int. | Schwingung |
| 3420 $cm^{-1}$ | sb | O-H Val.-Schwg. |
| 2990 $cm^{-1}$ | s | C-H Val.-Schw. |
| 1160 $cm^{-1}$ | s | -C-O-C- |
| 1070 $cm^{-1}$ | s | Acetal |
| 960 $cm^{-1}$ | s | -CH=CH- (trans, im Ring) |

| $^1$H-NMR-Spektrum (300,13 MHz) in $CDCl_3$ | | | | | |
|---|---|---|---|---|---|
| 0,71 | ppm | s | 3 | H | -$CH_3$ (C-18) |
| 0,98 | ppm | d | 3 | H | -$CH_3$ (C-21) $^3$J: 6,6 Hz |
| 1,01 | ppm | s | 3 | H | -$CH_3$ (C-19) |
| 0,87 - 2,53 | ppm | m | 24 | H | nicht zugeordnete Protonen |
| 3,45 - 3,58 | ppm | m | 1 | H | 3α-H |
| 3,83 - 4,02 | ppm | m | 4 | H | -O-$CH_2CH_2$-O- |
| 4,865 | ppm | t | 1 | H | -CH- (C-25) $^3$J: 4,9 Hz |
| 5,21 - 5,38 | ppm | m | 3 | H | =CH- (C-6, C-22, C-23) |

| Optische Drehwerte (c = 1,6613 in $CHCl_3$; T = 25 °C) | | |
|---|---|---|
| λ | α | [α] |
| 589 nm | -1,014 | -28,8 |
| 578 nm | -1,056 | -30,0 |
| 546 nm | -1,199 | -34,0 |
| 436 nm | -1,990 | -56,5 |
| 365 nm | nicht meßbar | |

| $^{13}$C-NMR-Spektrum (75,47 MHz) in $CDCl_3$ | | | |
|---|---|---|---|
| 37,25 | ppm t C-1 | 55,9 | ppm d C-14 |
| 31,6 | ppm t C-2 | 24,25 | ppm t C-15 |
| 71,7 | ppm d C-3 | 28,0 | ppm t C-16 |
| 42,25 | ppm t C-4 | 56,75 | ppm d C-17 |
| 140,7 | ppm s C-5 | 12,2 | ppm q C-18 |
| 139,6 | ppm d C-6 | 19,4 | ppm d C-19 |
| 31,83 | ppm t C-7 | 34,5 | ppm d C-20 |
| 31,85 | ppm d C-8 | 20,5 | ppm d C-21 |
| 50,1 | ppm d C-9 | 119,2 | ppm d C-22 |
| 36,5 | ppm s C-10 | 121,5 | ppm d C-23 |
| 21,00 | ppm t C-11 | 32,6 | ppm t C-24 |
| 39,7 | ppm t C-12 | 104,12 | ppm d C-25 |
| 31,86 | ppm s C-13 | 64,9 | ppm t $-O-CH_2CH_2-O-$ |

Massenspektrum (MAT, 70 eV, T: 90°C)
$M^+$(ber.): 414,3134 g/Mol

| Masse | I(rel.) | Fragment |
|---|---|---|
| m/e = 414 | 0,2 % | $M^+$ |
| m/e = 413 | 7,1 % | $M^+$ -H |
| m/e = 396 | 2,1 % | $M^+$ $-H_2O$ |
| m/e = 395 | 10,2 % | $M^+$ -H, $-H_2O$ |
| m/e = 341 | 8,4 % | $M^+$ - 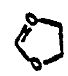 |
| m/e = 301 | 5,3 % | $M^+$ - 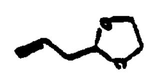 |
| m/e = 283 | 2,3 % | $M^+$ - 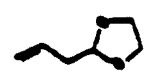 |
| m/e = 73 | 100,0 % | 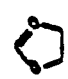 |

**Beispiel 6**

3β-Hydroxy-26,27-bisnor-cholesta-5,23t-dien-25-al (IV)

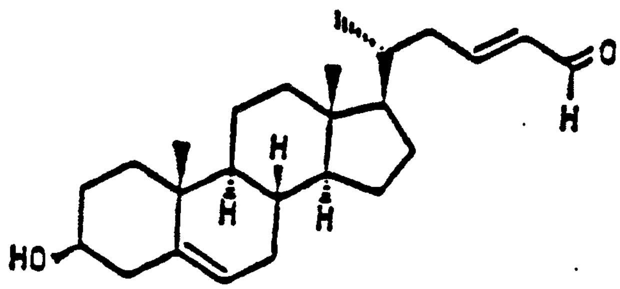

IV

Summenformel: $C_{25}H_{38}O_2$ Molekulargewicht: 370,58 g/Mol

a): 4,29 g (0,01 Mol) 3α,5α-Cyclo-6β-methoxy-24-(1′,3′-dioxolan-2′-yl)-chol-22t-en (V) werden in 180 ml Aceton gelöst, mit einer Lösung von 1,90 g (0,01 Mol) p-Toluolsulfonsäure in 70 ml Wasser versetzt und 24 h unter Rückfluß erhitzt. Nach Abziehen des Acetons wird mit Essigester versetzt. Die Essigester-Lösung des Aldehyds IV wird zunächst mit gesättigter Natriumhydrogencarbonat-Lösung säurefrei gewaschen und anschließend mit 50 ml Wasser und 50 ml gesättigter Natriumchlorid-Lösung ausgeschüttelt. Nach Trocknen über wasserfreiem Natriumsulfat filtriert man das Trockenmittel ab und entfernt das Lösungsmittel am Rotationsverdampfer bei vermindertem Druck (ca. 12 Torr); eine 30-minütige Nachtrocknung im Vakuum (0,1 Torr) schließt sich an. Das erhaltene Produkt ist für die folgenden Reaktionen ausreichend rein.
Ausbeute: 3,41 g (0,0092 Mol; 92 % d.Th.) IV

b): 4,15 g (0,01 Mol) 3β-Hydroxy-24-(1′,3′-dioxolan-2′-yl)-chola-5,22c-dien (II) werden in 150 ml Aceton gelöst, mit einer Lösung von 1,60 g (0,008 Mol) p-Toluolsulfonsäure in 80 ml Wasser versetzt und 16 h unter Rückfluß erhitzt. Nach Abziehen des Acetons wird mit Essigester versetzt und wie unter a) beschrieben aufgearbeitet. Das erhaltene Produkt ist für die folgenden Reaktionen ausreichend rein.
Ausbeute: 3,56 g (0,0096 Mol; 96 % d.Th.) IV

Eine analytische Probe erhält man durch PSC (Kieselgel; Cyclohexan/Essigester : 2/l; $R_f$: 0,38-0,45) und Umkristallisation aus Diethylether. Fp: 108-109 °C (Diethylether)

| IR-Spektrum (KBr-Preßling) | | |
|---|---|---|
| | Int. | Schwingung |
| 3350 $cm^{-1}$ | sb | -OH Val.-Schw. |
| 2900 $cm^{-1}$ | s | -CH Val.-Schw. |
| 1710 $cm^{-1}$ | s | -CHO Val.-Schw. |
| 995 $cm^{-1}$ | s | C=C Def.-Schw. (Seitenkette) |
| 835 $cm^{-1}$ | s | C=C Def.-Schw. (B-Ring) |

| Massenspektrum (MAT, 70 eV, T: 110 °C) | | |
|---|---|---|
| $M^+$(ber.): 370,2872 g/Mol | | |
| Masse | I(rel.) | Fragment |
| m/e = 370 | 19,0 % | $M^+$ |
| m/e = 352 | 11,8 % | $M^+$ -$H_2O$ |
| m/e = 337 | 8,4 % | $M^+$ -$H_2O$, - $CH_3$ |
| m/e = 69 | 18,1 % | $CH_2$-CH=CH-CHO |
| m/e = 57 | 100,0 % | $H_2C$=CH-CH=OH (A-Ring-Fragment) |

| $^{13}C$-NMR-Spektrum (75,47 MHz) in $CDCl_3$ | | | |
|---|---|---|---|
| 37,2 | ppm t C-1 | 56,6 | ppm d C-14 |
| 31,78 | ppm t C-2* | 24,2 | ppm t C-15 |
| 71,6 | ppm d C-3 | 28,2 | ppm t C-16 |
| 42,2 | ppm t C-4 | 55,7 | ppm d C-17 |
| 140,8 | ppm s C-5 | 11,8 | ppm q C-18 |
| 121,4 | ppm d C-6 | 19,0 | ppm q C-19 |
| 31,5 | ppm t C-7* | 35,7 | ppm d C-20 |
| 31,84 | ppm d C-8 | 19,3 | ppm q C-21 |
| 50,0 | ppm d C-9 | 39,54 | ppm t C-22** |
| 36,4 | ppm s C-10 | 134,3 | ppm d C-23 |
| 21,0 | ppm t C-11 | 157,9 | ppm d C-24 |
| 39,57 | ppm t C-12** | 193,9 | ppm d C-25 |
| 42,4 | ppm s C-13 | | |

| $^{1}H$-NMR-Spektrum (300,13 MHz) in $CDCl_3$ | | | | | |
|---|---|---|---|---|---|
| 0,71 | ppm | s | 3 | H | $-CH_3$ (C-18) |
| 0,98 | ppm | d | 3 | H | $-CH_3$ (C-21) $^3J$: 6,7 Hz |
| 1,01 | ppm | s | 3 | H | $-CH_3$ (C-19) |
| 0,82 - 2,33 | ppm | m | 20 | H | nicht zugeordnete Protonen |
| 2,39 - 2,485 | ppm | m | 2 | H | -CH-CHH-CH=CH-CHO |
| 3,47 - 3,57 | ppm | m | 1 | H | -CH(OH)- |
| 5,34 | ppm | m | 1 | H | -C=CH- (C-6) |
| 6,12 | ppm | dddd | 1 | H | =CH-CHO<br>$^3J$: 15,5 Hz $^3J$: 7,9 Hz<br>$^4J$: 1,0 Hz $^4J$: 0,4 Hz |
| 6,85 | ppm | ddd | 1 | H | -CH=CH-CHO $^3J$: 8,7 Hz<br>$^3J$: 15,5 Hz $^3J$: 6,2 Hz |
| 9,50 | ppm | d | 1 | H | -CHO $^3J$: 7,9 Hz |

| Optische Drehwerte (c = 2,3814 in $CHCl_3$; T = 25 °C) | | |
|---|---|---|
| λ | α | [α] |
| 589 nm | -0,922 | -38,7 |
| 578 nm | -0,964 | -40,5 |
| 546 nm | -1,096 | -46,0 |
| 436 nm | -1,836 | -77,1 |
| 365 nm | - nicht meßbar | |

| Elementar-Analyse | | |
|---|---|---|
| | berechnet | gefunden |
| C | 81,03 % | 81,0 % |
| H | 10,34 % | 10,4 % |
| X | 8,63 % | 8,6 % |

**Beispiel 7**

3β-Hydroxy-chola-5,23t-dien-24-carbonsäure III

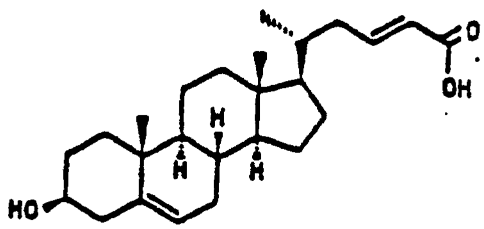

III

**Summenformel: $C_{25}H_{38}O_3$ Molekulargewicht: 386,58 g/Mol**

Eine Lösung von 3,71 g (0,01 Mol) 3β-Hydroxy-26,27-bisnor-cholesta-5,23t-dien-25-al (IV) in 55 ml t-Butanol wird mit 2,95 g (9,4 mMol) 2-Methyl-2-buten versetzt. Zu dieser Lösung addiert man 1,86 g (16,4 mMol) Natriumchlorit (80 %, tech.) in 7 ml Wasser und 4,82 g (35 mMol) Natriumdihydrogenphosphat in 6 ml Wasser; anschließend wird 48 h bei Zimmertemperatur gerührt. Zur Aufarbeitung versetzt man die Suspension mit 5 %iger Salzsäure bis zur deutlich sauren Reaktion. Die organischen Lösungsmittel werden unter vermindertem Druck (12 Torr) am Rotationsverdampfer entfernt; den resultierenden wäßrigen Rückstand extrahiert man mit 100 ml Essigester. Nach Waschen der abgetrennten organischen Phase mit 100 ml Wasser, 50 ml Natriumhydrogencarbonat-Lösung und 50 ml Natriumchlorid-Lösung trocknet man über wasserfreiem Magnesiumsulfat. Das Trockenmittel wird abfiltriert und das organische Lösungsmittel wird zunächst am Rotationsverdampfer (bei ca. 12 Torr) danach im Vakuum bei 0,1 Torr entfernt. Das isolierte Rohprodukt ist für die weiteren Umsetzungen ausreichend rein.
Ausbeute: 3,64 g (0,0094 Mol, 94 % d.Th.)
3β-Hydroxy-chola-5,23t-dien-24-carbonsäure (III)

Eine analytische Probe erhält man durch PSC (Kieselgel; Cyclohexan/Essigester = 1/l; $R_f$: 0,22-0,45) und Umkristallisation aus Essigester.
Fp: 98-101 °C (Essigester)

| Elementar-Analyse | | |
|---|---|---|
| | berechnet | gefunden |
| C | 77,68 % | 77,6 % |
| H | 9,91 % | 9,9 % |
| X | 12,41 % | 12,5 % |

| IR-Spektrum (KBr-Preßling) | | |
|---|---|---|
| | Int. | Schwingung |
| 3360 $cm^{-1}$ | s | -OH Val.-Schw. |
| 2940 $cm^{-1}$ | s | -CH Val.-Schw. |
| 2655 $cm^{-1}$ | s | -OH Val.-Schw. (assoziiert) |
| 1690 $cm^{-1}$ | s | -C=O Val.-Schw. |
| 1460 $cm^{-1}$ | m | $-CH_2-$ Def.-Schw. |
| 1390 $cm^{-1}$ | m | $-CH_3-$ Def.-Schw. |
| 1050 $cm^{-1}$ | s | -C-OH- Def.-Schw. |

| Massenspektrum (CH-7, 70 eV, T: 100 °C) | | |
|---|---|---|
| $M^+$ (ber.): 386,2821 g/Mol | | |
| Masse | I(rel.) | Fragment |
| m/e = 386 | 6 % | $M^+$ |
| m/e = 368 | 15 % | $M^+$ $-H_2O$ |
| m/e = 353 | 12 % | $M^+$ $-H_2O$, $-CH_3$ |
| m/e = 328 | 15 % | $M^+$ -CH-COOH |
| m/e = 301 | 13 % | $M^+$ $-CH_2CH=CH-COOH$ |
| m/e = 273 | 6 % | $M^+$ $-CH_3CHCH_2CH=CH-COOH$ |
| m/e = 43 | 100 % | ? |

| $^1H$-NMR-Spektrum (300,13 MHz) in DMSO-$d_6$ | | | | | |
|---|---|---|---|---|---|
| 0,67 | ppm | s | 3 | H | $-CH_3$ (C-18) |
| 0,91 | ppm | d | 3 | H | $-CH_3$ (C-21) |
| 0,95 | ppm | s | 3 | H | $-CH_3$ (C-19) |
| 0,69 - 2,32 | ppm | m | 23 | H | nicht zugeordnete Protonen |
| 3,27 | ppm | m | 1 | H | -CH(OH)- |
| 4,60 | ppm | sb | 1 | H | -O$\overline{H}$ (Alkohol) |
| 5,25 | ppm | m | 1 | H | -C=CH- |
| 5,73 | ppm | d | 1 | H | =CH-COOH |
| 6,78 | ppm | ddd | 1 | H | =C$\overline{H}$=CH-COOH |
| 12,03 | ppm | sb | 1 | H | =C$\overline{O}$OH |

| $^{13}$C-NMR-Spektrum (75,47 MHz) in DMSO-$d_6$ / Methanol-$d_4$ T: 35° C | | | |
|---|---|---|---|
| 37,0 | ppm t C-1 | 56,2 | ppm d C-14 |
| 31,3 | ppm t C-2* | 23,9 | ppm t C-15 |
| 70,0 | ppm d C-3 | 27,6 | ppm t C-16 |
| 42,2 | ppm t C-4 | 55,3 | ppm d C-17 |
| 141,1 | ppm s C-5 | 11,6 | ppm q C-18 |
| 120,3 | ppm d C-6 | 19,1 | ppm q C-19 |
| 31,4 | ppm t C-7* | 35,1 | ppm d C-20 |
| 31,5 | ppm d C-8 | 18,7 | ppm q C-21 |
| 49,6 | ppm d C-9 | 42,2 | ppm t C-22 |
| 36,1 | ppm s C-10 | 123,3 | ppm d C-23 |
| 20,6 | ppm t C-11 | 147,0 | ppm d C-24 |
| 39,0 | ppm t C-12 | 166,9 | ppm s C-25 |
| 42,0 | ppm s C-13 | | |

| Optische Drehwerte (c = 2,0102 in Methanol, T = 25° C) | | |
|---|---|---|
| λ | α | [α] |
| 589 nm | -0,540 | -26,7 |
| 578 nm | -0,568 | -28,3 |
| 546 nm | -0,649 | -32,3 |
| 436 nm | -1,130 | -56,2 |
| 365 nm | nicht meßbar | |

**Beispiel 8**

1-(1′,3′-Dioxolan-2′-yl)-ethyl-triphenyl-phosphoniumbromid VIa

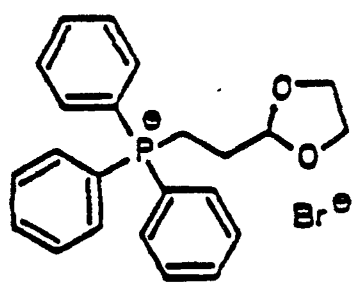

VI a

**Summenformel: $C_{23}H_{24}BrO_2P$ Molekulargewicht: 443.32 g/Mol**

In einem Lösungmittelgemisch aus 150 ml wasserfreiem Benzol und 60 ml wasserfreiem Cyclohexan erhitzt man 90.52 g (0,5 Mol) frisch destilliertes farbloses 2′-(2-Bromethyl)-1′,3′-dioxolan VIb und 196.7 g (0,75 Mol) Triphenylphosphin 60 h unter Rückfluß; das Phosphoniumsalz fällt dabei als schwach gelbes sehr zähes Öl aus. Nach Abkühlen des Reaktionsgemisches wird die benzolische Lösung von dem

Reaktionsprodukt abdekantiert und danach zur Reinigung noch zweimal mit je 100 ml heißem Benzol gewaschen.
Das Salz ist sehr stark hygroskopisch (zerfließt an der Luft !) und kristallisiert nach mehrtägigem Trocknen unter Vakuum im Exsikkator über Phosphorpentoxid. Man erhält sehr reines 2-(1′,3′-Dioxolan-2′-yl)-ethyl-triphenylphosphoniumbromid VIa als weißes Pulver.
Ausbeute 42.7 g (0.0962 Mol; 96.2 % d. Th.)

Eine weitere Reinigung ist infolge des sehr starken wasseranziehenden Verhaltens des Produktes nur schlecht möglich. Die Aufbewahrung sollte unter Vakuum im Exsikkator erfolgen.

Diese Synthese erfolgt analog der von J.C. Stowell und D.R. Keith in Synthesis [1979], Seite 132-133, Georg Thieme Publishers beschriebenen Herstellungsweise.

| IR-Spektrum (KBr-Preßling) | | |
|---|---|---|
| | Int. | Schwingung |
| 3005 $cm^{-1}$ | m | C-H Val.-Schwg. (aromatisch) |
| 2980 $cm^{-1}$ | s | C-H Val.-Schwg. (aliphatisch) |
| 960 $cm^{-1}$ | s | Acetal |
| 900 $cm^{-1}$ | s | mono-sub. Aromat |
| 730 $cm^{-1}$ | s | mono-sub. Aromat |

| $^1$H-NMR-Spektrum (300.13 MHz) in $CDCl_3$ | | | | | |
|---|---|---|---|---|---|
| 1,95 | ppm | m | 2 | H | $-CH_2-CH_2-CH-$ |
| 3,76 - 3,94 | ppm | m | 2 | H | $P^+-CH_2-$ |
| 3,90 - 4,09 | ppm | m | 4 | H | $-O-CH_2-CH_2-O-$ |
| 5,16 | ppm | t | 1 | H | -CH-O- $^3$J:4,1 Hz |
| 7,84 - 8,05 | ppm | m | 15 | H | =CH (aromatisch) |

| $^{13}$C-NMR-Spektrum (75,47 MHz) in $CDCl_3$ | | | |
|---|---|---|---|
| 26.3 | ppm t C-1 | 118,7 | ppm s =C-P* |
| 15.2 | ppm t C-2 | 130,1 | ppm d p-C (Aromat) |
| 101,9 | ppm d C-1′ | 133,6 | ppm d m-C (Aromat)* |
| 64.5 | ppm t C-3′ + C-4′ | 134.8 | ppm d o-C (Aromat)* |

## Erfindungsgemäße Beispiele

### Beispiel 9

3β-Hydroxy-chola-5,23t-dien-24-carbonsäure-methylester I

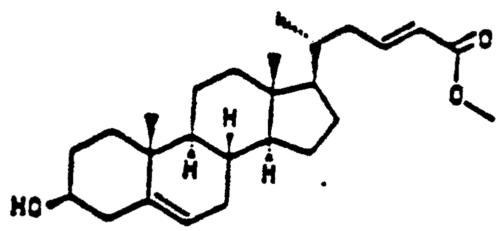

I

Summenformel: $C_{26}H_{40}O_3$ Molekulargewicht: 400.61 g/Mol

Eine Lösung von 8,72 g (0,02 Mol) 3β-Hydroxy-chola-5,23t-dien-24-carbonsäure (III) in 100 ml wasserfreiem THF wird bei 0° C unter Rühren mit einer etherischen Diazomethan-Lösung versetzt.

Nach einer Reaktionszeit von 3 min. wird überschüssiges Diazomethan bei 0° C durch Zugabe von 25%iger Essigsäure vernichtet (Lösung wird farblos). Nach Waschen mit gesättigter Natriumhydrogencarbonat-Lösung bis zur neutralen Reaktion, 50 ml Wasser und 50 ml gesättigter Natriumchlorid-Lösung trocknet man über wasserfreiem Magnesiumsulfat. Man filtriert das Trockenmittel ab und entfernt das THF unter vermindertem Druck zunächst am Rotationsverdampfer (12 Torr) und dann im Vakuum bei 0,1 Torr. Man erhält den für weitere Umsetzungen ausreichend reinen Methylester. PSC (Kieselgel, Cyclohexan/Essigester : 1/1; $R_f$: 0,80-0,89) liefert eine analytisch reine Probe.

Ausbeute: 7.66 g (0,019 Mol, 95% d. Th.) 3β-Hydroxy-chola-5,23t-dien-24-carbonsäure-methylester (I) als farbloses Öl, das bei längeren Stehenlassen langsam kristallisiert

Pf: 52 - 55° C (nach PSC)

| IR-Spektrum (KBr-Preßling) | | |
|---|---|---|
| | Int. | Schwingung |
| 3400 $cm^{-1}$ | s | O-H Val.-Schwg. |
| 2985 $cm^{-1}$ | s | C-H Val.-Schwg. |
| 1705 $cm^{-1}$ | s | C=O Val.-Schwg. |
| 1640 $cm^{-1}$ | m | $CH=CHCOOCH_3$ |
| 980 $cm^{-1}$ | m | -CH=CH- (trans) |
| 810 $cm^{-1}$ | m | -C=CH- (Ring) |

| $^1$H-NMR-Spektrum (300,13 MHz) in $CDCl_3$ | | | | | |
|---|---|---|---|---|---|
| 0,69 | ppm | s | 3 | H | $-CH_3$ (C-18) |
| 0,95 | ppm | d | 3 | H | $-CH_3$ (C-21) |
| 1,01 | ppm | s | 3 | H | $-CH_3$ (C-19) |
| 0,88 - 2,34 | ppm | m | 24 | H | nicht zugeordnete Protonen |
| 3,45 - 3,58 | ppm | m | 1 | H | 3-H |
| 3,73 | ppm | s | 3 | H | $-COOCH_3$ |
| 5,33 - 5,37 | ppm | m | 1 | H | =CH- (C-6) |
| 5,82 | ppm | ddd | 1 | H | $=CH-COOCH_3$ $^3J$: 15,6 Hz $^4J$: 1,1 Hz $^4J$: 0,9 Hz |
| 6,96 | ppm | ddd | 1 | H | $-CH=CHCOOCH_3$ $^3J$: 15,6 Hz $^3J$: 8,8 Hz $^3J$: 6,6 Hz |

| Massenspektrum (MAT, 70 eV, T: 110° C) | | |
|---|---|---|
| $M^+$(ber.): 400.2977 g/Mol | | |
| Masse | I(rel.) | Fragment |
| m/e = 400 | 100,0 % | $M^+$ |
| m/e = 385 | 17,6 % | $M^+$ -$CH_3$ |
| m/e = 382 | 75,1 % | $M^+$ -$H_2O$ |
| m/e = 369 | 16,1 % | $M^+$ -$OCH_3$ |
| m/e = 367 | 32,5 % | $M^+$ -$H_2O$, -$CH_3$ |
| m/e = 341 | 10,6 % | $M^+$ -$COOCH_3$ |
| m/e = 315 | 46,2 % | $M^+$ -CH=CH-$COOCH_3$ |
| m/e = 273 | 11,2 % | $M^+$ -CH($CH_3$)-$CH_2$CH=CH-$COOCH_3$ |

| Optische Drehwerte (c = 2,2062 in $CHCl_3$; T = 25° C) | | |
|---|---|---|
| λ | α | [α] |
| 589 nm | +0,200 | + 9,1 |
| 578 nm | +0,206 | + 9,3 |
| 546 nm | +0,223 | +10,1 |
| 436 nm | nicht meßbar | |
| 365 nm | nicht meßbar | |

| $^{13}$C-NMR-Spektrum (75,47 MHz) in $CDCl_3$ | | | |
|---|---|---|---|
| 37,2 | ppm t C-1 | 56,6 | ppm d C-14 |
| 31,85 | ppm t C-2 * | 24,3 | ppm t C-15 |
| 71,7 | ppm d C-3 | 28,2 | ppm t C-16 |
| 42,2 | ppm t C-4 | 55,5 | ppm d C-17 |
| 140,75 | ppm s C-5 | 11,9 | ppm q C-18 |
| 121,5 | ppm d C-6 | 19,0 | ppm q C-19 |
| 31,58 | ppm t C-7* | 36,5 | ppm d C-20 |
| 31,9 | ppm d C-8 | 19,4 | ppm q C-21 |
| 50,0 | ppm d C-9 | 39,05 | ppm t C-22 |
| 36,5 | ppm s C-10 | 148,5 | ppm d C-23 |
| 21,0 | ppm t C-11 | 122,1 | ppm d C-24 |
| 39,6 | ppm t C-12 | 167,0 | ppm s C-25 |
| 42,2 | ppm s C-13 | 51,4 | ppm q -O-$CH_3$ |

| Elementar-Analyse | | |
|---|---|---|
| | berechnet | gefunden |
| C | 77,95 % | 77,6 % |
| H | 10,06 % | 9,8 % |
| X | 11,99 % | 12,6 % |

Cholesterol-Biosynthese-Hemmung
Zellkultur: HEP-G2-Zellen
Lösungsmittel-Endkonzentration: 1 % DMSO

| Präparat-Konzentration (Mol/1) | Acetateinbau in Cholesterin im Vergleich zur Kontrolle |
|---|---|
| 2,5 x $10^{-4}$ | 6 % |
| 2,5 x $10^{-5}$ | 17 % |
| 1,0 x $10^{-6}$ | 52 % |
| 1,0 x $10^{-7}$ | 56 % |
| $IC_{50}$: ca. 5,0 x $10^{-7}$ Mol/1 | |

**Beispiel 10**

3β,25-Dihydroxy-26,27-bisnor-cholesta-5,23t-dien I

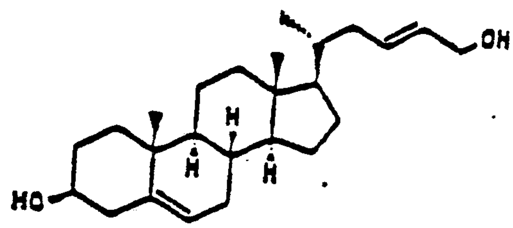

I

**Summenformel: $C_{25}H_{39}O_2$** **Molekulargewicht: 371,59 g/Mol**

Eine Lösung von 5,56 g (0,015 Mol) 3β-Hydroxy-26,27-bisnor-cholesta-5,23t-dien-25-al (IV) in 180 ml absolutem Ether wird mit 0,304 g (0,008 Mol) Lithiumaluminiumhydrid versetzt und 6 h auf Rückflußtemperatur erhitzt. Nach beendeter Reaktion wird bei 0 °C vorsichtig mit 5 %iger Ammoniumchlorid-Lösung hydrolysiert. Nach der Hydrolyse wird die Etherphase abgetrennt und nacheinander mit 40 ml gesättigter Natriumhydrogencarbonat-Lösung, mit 50 ml Wasser und mit 25 ml gesättigter Natriumchlorid-Lösung gewaschen; anschließend wird über Magnesiumsulfat getrocknet. Zur Isolierung des Reaktionsprodukts filtriert man vom Magnesiumsulfat ab, entfernt das Lösungmittel zunächst am Rotationsverdampfer unter vermindertem Druck (12 Torr) und anschließend im Vakuum bei 0,1 Torr. Das erhaltene Produkt wird aus Hexan/Ether umkristallisiert. Eine Reinigung mittels PSC ist möglich (Kieselgel; Cyclohexan/Essigester:1/1, $R_f$: 0,32-0,45).

Ausbeute: 4,68 g (0,0126 Mol; 85 % d. Th.)

3β,25-Dihydroxy-26,27-bis-nor-cholesta-5,23t-dien (I) Fp: 166 - 167 °C (Essigester/Cyclohexan)

Der Alkohol I kann auch ausgehend von 3β-Hydroxy-24-carboxymethyl-chola-5,23t-dien I synthetisiert werden:

4,01 g (0,01 Mol) 3β-Hydroxy-24-carboxymethyl-chola-5,23t-dien werden in 80 ml wasserfreiem THF gelöst, mit 253 mg (6,66 mMol) Lithiumaluminiumhydrid versetzt und 6,5 h unter Rückfluß erhitzt. Nach beendeter Reduktion läßt man den Ansatz auf Raumtemperatur kommen, hydrolysiert mit 25 ml 5%iger Salzsäure, versetzt mit 100 ml Ether und trennt die organische Phase ab. Zur weiteren Aufarbeitung wird die abgetrennte Phase zunächst mit gesättigter Natriumhydrogencarbonatlösung säurefrei und anschließend mit 50 ml Wasser und 50 ml gesättigter Natriumchlorid-Lösung gewaschen. Nach Trocknen über wasserfreiem Magnesiumsulfat entfernt man das Trocknungsmittel. Die organischen Lösungsmittel werden am Rotationsverdampfer abdestilliert; eine Nachtrocknung im Vakuum schließt sich an. Das reine Diol erhält man durch PSC (Kieselgel, Cyclohexan/Essigester: 1/1, $R_f$: 0,32-0,45). Ausbeute: 2,90 g (7,8 mMol; 78 % d.Th) 3β,25-Dihydroxy-26,27-bisnor-cholesta-5,23t-dien (I)

| IR-Spektrum (KBr-Preßling) | | |
|---|---|---|
| | Int. | Schwingung |
| 3350 $cm^{-1}$ | sb | OH Val.-Schw. |
| 2950 $cm^{-1}$ | s | C-H Val.-Schw. |
| 1460 $cm^{-1}$ | s | $CH_2$ Def.-Schw. |
| 1380 $cm^{-1}$ | s | $CH_3$ Def.-Schw. |
| 1050 $cm^{-1}$ | s | -C-OH Def.-Schw. |
| 800 $cm^{-1}$ | s | -C=C- Def.-Schw. |

| $^1$H-NMR-Spektrum (300,13 MHz) IN $CDCl_3$ | | | | | |
|---|---|---|---|---|---|
| 0,69 | ppm | s | 3 | H | $-CH_3$ (C-18) |
| 0,93 | ppm | d | 3 | H | $-CH_3$ (C-21) $^3$J: 6,6 Hz |
| 1,01 | ppm | s | 3 | H | $-CH_3$ (C-19) |
| 0,79 - 1,01 | ppm | m | 22 | H | nicht zugeordnete Protonen |
| 1,70 | ppm | sb | 2 | H | -OH |
| 3,47 - 3,57 | ppm | m | 1 | H | -CH(OH)- |
| 4,10 | ppm | d | 2 | H | $=\bar{C}H-CH_2-OH$ |
| 5,35 | ppm | m | 1 | H | =CH- ($\bar{C}$-6) |
| 5,59 - 5,72 | ppm | m | 2 | H | -CH=CH- |

| $^{13}$C-NMR-Spektrum (75,47 MHz) in Methanol-$d_4$ | | | |
|---|---|---|---|
| 37,9 | ppm t C-1 | 57,3 | ppm d C-14 |
| 31,8 | ppm t C-2 | 24,7 | ppm t C-15 |
| 71,4 | ppm d C-3 | 28,6 | ppm t C-16 |
| 42,6 | ppm t C-4 | 56,3 | ppm d C-17 |
| 141,7 | ppm s C-5 | 12.1 | ppm q C-18 |
| 121,6 | ppm d C-6 | 19,6 | ppm q C-19 |
| 32,4 | ppm t C-7 | 36,6 | ppm d C-20 |
| 32,3 | ppm d C-8 | 19,0 | ppm q C-21 |
| 50,5 | ppm d C-9 | 40,3 | ppm t C-22 |
| 37,0 | ppm s C-10 | 130,2 | ppm d C-23 |
| 21,6 | ppm t C-11 | 131,7 | ppm d C-24 |
| 39,5 | ppm t C-12 | 62,9 | ppm t C-25 |
| 42,8 | ppm s C-13 | | |

| Elementar-Analyse | | |
|---|---|---|
| | berechnet | gefunden |
| C | 80,81 % | 80,7 % |
| H | 10,58 % | 10,5 % |
| X | 8,61 % | 8,9 % |

| Massenspektrum (MAT, 70 eV, T: 130 ° C) | | |
|---|---|---|
| $M^+$(ber.): 371,2950 g/Mol | | |
| Masse | I(rel.) | Fragment |
| m/e = 371 | 60,6 % | $M^+$ |
| m/e = 354 | 44,4 % | $M^+$ -$H_2O$ |
| m/e = 339 | 18,4 % | $M^+$ -$H_2O$,-$CH_3$ |
| m/e = 301 | 42,6 % | $M^+$ -$CH_2$-CH=CH-$CH_2OH$ |
| m/e = 283 | 70,4 % | $M^+$ -$H_2O$,-$CH_2$-CH=CH-$CH_2OH$ |
| m/e = 81 | 100,0 % | $M^+$ ? |

| Optische Drehwerte (c = 2,9404 in Methanol, T=25 ° C) | | |
|---|---|---|
| λ | α | [α] |
| 589 nm | -1,101 | -37,4 |
| 578 nm | -1,155 | -39,3 |
| 546 nm | -1,317 | -44,8 |
| 436 nm | -2,282 | -77,6 |
| 365 nm | nicht meßbar | |

Cholesterol-Biosynthese-Hemmung
Zellkultur: HEP-G2-Zellen
Lösungsmittel-Endkonzentration: 1 % DMSO

| Präparat-Konzentration (Mol/1) | Acetateinbau in Cholesterin im Vergleich zur Kontrolle |
|---|---|
| 2,5 x $10^{-4}$ | 18 % |
| 2,5 x $10^{-5}$ | 26 % |
| 1,0 x $10^{-6}$ | 88 % |
| 1,0 x $10^{-7}$ | 76 % |
| $IC_{50}$: ca. 2,0 x $10^{-6}$ Mol/1 | |

## Ansprüche

1. Verbindung I

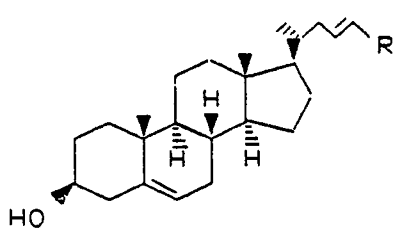

I

mit R gleich -COOX oder $CH_2OH$, wobei
X Wasserstoff oder ($C_1$-$C_8$)-Alkyl, geradkettig oder verzweigt, bedeutet.

2. Verbindung nach Anspruch 1, dadurch gekennzeichnet, daß X ($C_1$-$C_4$)-Alkyl bedeutet.

3. Verbindung nach Anspruch 1, dadurch gekennzeichnet, daß X $CH_3$ bedeutet.

4. Verbindung nach Anspruch 1, dadurch gekennzeichnet, daß R $CH_2OH$ bedeutet.

5. Verfahren zum Herstellen von Verbindungen I nach Anspruch 1, dadurch gekennzeichnet, daß man eine Verbindung III

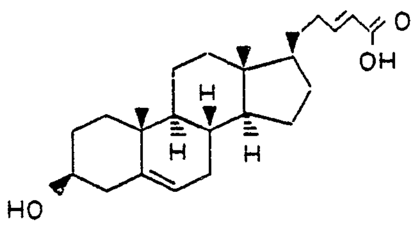

III

zu I verestert.

6. Verwendung einer Verbindung I nach Anspruch 1 als Arzneimittel zur Prophylaxe und Therapie von Hypercholesterinämie.

7. Verwendung einer Verbindung I nach Anspruch 1 zur Herstellung eines Arzneimittels zur Prophylaxe und Therapie der Hypercholesterinämie.

Patentansprüche für folgende Vertragsstaaten: ES, GR

1. Verfahren zum Herstellen einer Verbindung I

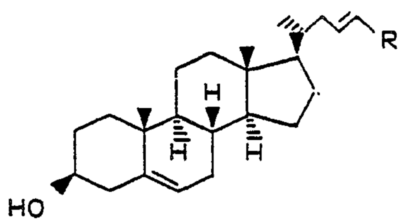

I

mit R gleich -COOX oder $CH_2OH$, wobei
X Wasserstoff oder ($C_1$-$C_8$)-Alkyl, geradkettig cder verzweigt, bedeutet,
dadurch gekennzeichnet, daß man eine Verbindung III

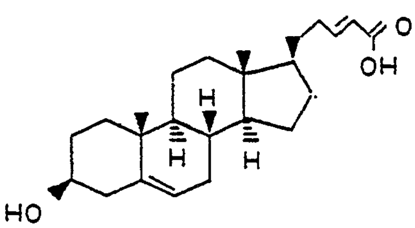

III

zu I verestert.

2. Verfahren nach Anspruch 1, dadurch gekennzeichnet, daß X ($C_1$-$C_4$)-Alkyl bedeutet.

3. Verfahren nach Anspruch 1, dadurch gekennzeichnet, daß X $CH_3$ bedeutet.

4. Verfahren nach Anspruch 1, dadurch gekennzeichnet, daß R $CH_2OH$ bedeutet.

5. Verwendung einer Verbindung I nach Anspruch 1 als Arzneimittel zur Prophylaxe und Therapie von Hypercholesterinämie.

6. Verwendung einer Verbindung I nach Anspruch 1 zur Herstellung eines Arzneimittels zur Prophylaxe und Therapie der Hypercholesterinämie.